# DEMANDE DE BREVET EUROPEEN

(11) **EP 3 539 455 A1**
(43) Date de publication de la demande: **18.09.2019**
(21) Numéro de dépôt: 18305275.2
(22) Date de dépôt: 14.03.2018
(51) Int. Cl.: A61B 1/04, A61B 1/00, G06F 17/30

(54) **PROCÉDÉ POUR DÉTERMINER AUTOMATIQUEMENT LA QUALITÉ DE VISUALISATION DES IMAGES EN VIDÉOCAPSULE ENDOSCOPIQUE**

(71) Demandeur: Sorbonne Université, 75006 Paris (FR); ASSISTANCE PUBLIQUE, HOPITAUX DE PARIS, 75004 Paris (FR); Université Cergy-Pontoise, 95000 Cergy (FR); Centre National de la Recherche Scientifique, 75016 Paris (FR); ENSEA - Ecole Nationale Supérieure de l'Electronique et de ses Applications, 95800 Cergy (FR)
(72) Inventeur: DRAY, Xavier, 75012 PARIS (FR); HISTACE, Aymeric, 95180 Menucourt (FR)
(74) Mandataire: A.P.I. Conseil

(57) **Abrégé**

L'invention concerne un procédé de réalisation d'un classifieur numérique d'images, afin de déterminer automatiquement la qualité de visualisation des images de vidéocapsule endoscopique de segments du tube digestif,
comprenant une étape d'acquisition vidéo dans le tube digestif par une vidéocapsule ; une étape d'extraction d'images de la vidéo; une étape dite « vérité terrain » d'évaluation clinique de la qualité de visualisation des images à partir de critères médicaux,
une étape de sélection d'un ensemble initial d'images avec visualisation «adéquate» et d'images avec visualisation «inadéquate», une étape de calcul d'au moins un paramètre numérique qui a trait à au moins l'un des critères médicaux, une étape d'apprentissage statistique automatique pour réaliser le classifieur numérique.

## Description

### DOMAINE DE L'INVENTION

La présente invention concerne un procédé pour déterminer la qualité de visualisation des images (images fixes ou vidéos) de vidéocapsule endoscopique (VCE), d'un ou plusieurs segments du tube digestif (intestin grêle, côlon, oesophage ou estomac).

### ETAT DE LA TECHNIQUE

La VCE est une méthode de première ligne dans l'exploration de l'intestin grêle, du fait notamment de son très bon profil de sécurité et de tolérance (examen ambulatoire), du caractère le plus souvent complet de l'examen (dans plus de 90% des cas), et de sa bonne rentabilité diagnostique (de l'ordre de 40%, toutes indications confondues) (1).

Depuis les années 2000, les dispositifs se sont diversifiés, des avancées technologiques importantes ont émergé. La capsule PillCam SB3® est la 3ème génération de capsule du grêle du constructeur Medtronic. Elle est la plus largement répandue, mais une concurrence importante a émergé, avec actuellement trois autres dispositifs de VCE du grêle commercialisés en France : Mirocam® (Intromedic), Endocapsule® (Olympus) et Capsocam® SV2 (Capsovision). Les améliorations technologiques proposées au cours des deux dernières décennies incluent notamment une meilleure résolution d'image, un nombre accru d'images captées (et éventuellement adapté à la vitesse de déplacement de la capsule dans l'intestin), un élargissement du champ de vision, une plus grande autonomie énergétique, des aides informatiques à la détection et la caractérisation des images pathologiques. Ainsi, la capsule SB de troisième génération a un taux d'images captées de 2 à 6 par seconde, plus élevé que celui de la SB de deuxième génération.

Malgré les améliorations technologiques, la rentabilité de cet examen peut être diminuée par une mauvaise visualisation de la muqueuse due à la présence de résidus alimentaires, de bulles ou de bile dans la lumière digestive. En effet, la capsule n'a aucune option de lavage ou d'aspiration du contenu digestif, susceptible de gêner la visualisation, comme il en existe en vidéo endoscopie. Il a été suggéré par une méta-analyse incluant six essais prospectifs et six essais rétrospectifs qu'une préparation intestinale par polyéthylène glycol (PEG, 2 à 4 litres la veille) ou phosphate de sodium permettait d'obtenir une meilleure visualisation de la muqueuse et un meilleur rendement diagnostique (2). Les recommandations se sont basées sur cette méta-analyse, conseillant la prise de PEG, sans en préciser formellement le volume (3).

Depuis la rédaction de ces recommandations, une étude multicentrique française (PREPINTEST, menée par le CHU de Brest), a randomisé 858 patients dans 3 bras de préparation différente (régime seul, régime avec préparation par 2000 ml de PEG la veille, et régime avec 500 ml de PEG une demi-heure après ingestion de la capsule). La rentabilité de l'examen était de l'ordre de 40% dans les 3 groupes, mais les scores de qualité de visualisation de la muqueuse étaient significativement améliorés par la prise de PEG (qu'elles qu'en soient les modalités). La durée de transit de la capsule dans l'intestin était significativement diminuée par la prise de PEG (qu'elles qu'en soient les modalités). La prise de PEG lors d'une VCE du grêle n'a pas un effet démontré sur la rentabilité de l'examen, mais semble améliorer les conditions de lecture et le degré de confiance avec lequel un opérateur rendra ses conclusions de l'examen.

L'une des raisons majeures pour lesquelles il est difficile de recommander formellement une méthode de préparation du grêle, ou de conclure en pratique clinique à la qualité d'un examen, est qu'il n'existe pas de score correctement validé pour évaluer la qualité de visualisation de la muqueuse intestinale, à l'instar du score de Boston pour la qualité de la préparation lors d'une coloscopie.

Un tel score est difficile à établir sur une base clinique, car une séquence complète du grêle comporte des milliers d'images, certaines propres, certaines sales, dans des proportions éminemment variables, avec en conséquence une reproductibilité médiocre d'un évaluateur à l'autre, sans qu'une "vérité" concernant la qualité de visualisation d'une image (et a fortiori d'une séquence vidéo) puisse être formellement établie.

Pour pallier la difficulté d'établir dans ce domaine une « vérité terrain » concernant la qualité de visualisation d'une image de l'intestin grêle, les inventeurs ont considéré tout d'abord important de poser la définition de ce qu'est une qualité de visualisation adéquate pour comparer les modalités de celle-ci. On comprend ici par « qualité de visualisation » la capacité (pour un lecteur formé à l'interprétation de l'examen) à voir correctement la surface muqueuse intestinale, notamment en ce qui concerne sa quantité (c'est à dire surface muqueuse non recouverte de liquides colorés ou de résidus solides ou de bulles ou de toute autre obstacle) et en ce qui concerne sa qualité (c'est à dire une muqueuse examinée avec des couleurs correctement rendues et une luminosité adéquate - ni trop sombre, ni trop surexposée).

Il sera par la suite décrit des images dites avec visualisation «adéquate» (dont la qualité de visualisation de la muqueuse est bonne) et des images dites avec visualisation «inadéquate» (dont la qualité de visualisation de la muqueuse est mauvaise).

Des méthodes d'évaluation ont été proposées afin de conclure à une qualité de visualisation acceptable de l'examen.

Ces échelles d'évaluation sont diverses :
- évaluation qualitative globale;
- évaluation qualitative plus détaillée intégrant la visibilité de la muqueuse, la présence de bulles ou débris ou le caractère sombre de l'image (5,6) ;
- évaluation quantitative mesurant le pourcentage de muqueuse visible, la quantité de bulles, débris, bile, chyme obstruant la lumière digestive (4,7,8).

Un des scores les plus complets, reproductible et facile à utiliser pour la VCE du grêle est celui proposé par Brotz et al. (4) Dans cette étude prospective, randomisée, trois échelles d'évaluation étaient comparées : une échelle qualitative globale (propreté adéquate versus inadéquate), une échelle qualitative (propreté mauvaise, modérée, bonne, excellente) et une échelle quantitative sur 10 points, avec 5 items entre 0 et 2 points (pourcentage de muqueuse visualisée, abondance de fluides/débris, des bulles, de bile/chyme, luminosité). Il y avait une association forte et significative entre ces trois scores avec une meilleure corrélation intra et inter observateurs pour le score quantitatif. Si ce score quantitatif n'est pas formellement validé, il correspond à celui qui est le plus largement utilisé.

Cependant, la méthode est lourde à mettre en oeuvre car nécessite une analyse complète par le praticien et prend du temps.

En parallèle du développement de score clinique de qualité de visualisation de la muqueuse intestinale lors d'examen par VCE, certains auteurs ont proposé une évaluation électronique. En effet, la visualisation de qualité des images par un examinateur est difficile car l'ensemble de la séquence contient des dizaines de milliers d'images. Certains auteurs ont donc développé des algorithmes informatiques permettant une approche automatisée et reproductible de cette question.

Ainsi, un concept de score colorimétrique électronique a été proposé par Van Weyenberg et al. (9) se basant l'analyse du ratio de pixels rouges/verts de la barre de défilement du logiciel de lecture des capsules SB (logiciel Rapid, système Pillcam®, Medtronic), une série d'images propre se reflétant par une zone plus rouge que verte dans la barre de défilement.

Cette approche a été affinée à l'échelle de chaque image individuelle dans la publication « Development and Validation of a Highly Sensitive and Highly Specific Computed Assessment of Cleansing Score for Small Bowel Capsule Endoscopy » United European Gastroenterology Week. 2016.(10). Abou Ali et al. (10) ont déterminé et validé un score appelé ICQPI (indice colorimétrique de la qualité de la préparation intestinale), basé sur le ratio rouges/verts (ratio R/V) d'images fixes, avec de bonnes performances diagnostiques (sensibilité à 91%, spécificité à 91%) pour un ratio > 1,6, validé sur des capsules PillCam® de 2ème génération, et avec pour référence une quadruple lecture, à l'insu, par des lecteurs experts.

### EXPOSE DE L'INVENTION

Un but de l'invention est de proposer une méthode d'évaluation de la qualité de visualisation d'un ou de plusieurs segments du tube digestif, notamment de la muqueuse intestinale ou du côlon lors d'examen par VCE.

L'invention repose sur une analyse automatique par un ordinateur, image par image, plus performante et mieux reproductible qu'une évaluation humaine dépendant de l'opérateur.

A cet effet, un premier aspect de l'invention propose un procédé de réalisation d'un classifieur numérique d'images déterminant automatiquement la qualité de visualisation des images de vidéocapsule endoscopique dans au moins un segment du tube digestif d'une personne, comprenant les étapes suivantes :
- une étape d'acquisition vidéo dans le segment du tube digestif par une vidéocapsule ;
- une étape d'extraction d'images de la vidéo;
- une étape dite « vérité terrain » d'évaluation clinique de la qualité de visualisation des images par un score, déterminé par une analyse visuelle des images, à partir d'un ou plusieurs critères médicaux,
pour répartir ces images, en fonction du résultat du score : en images dites avec visualisation «adéquate» et en images dites avec visualisation «inadéquate» ;
les critères médicaux étant choisis parmi la liste suivante : le pourcentage de muqueuse visualisée, la luminosité de l'image, l'abondance des bulles, la présence de bile/chyme, la présence de liquides et de débris non digérés,
- une étape de sélection d'un ensemble initial d'images parmi les images dites avec visualisation «adéquate» et les images dites avec visualisation «inadéquate»,
- une étape de calcul d'au moins un paramètre numérique qui a trait à au moins l'un des critères médicaux de l'étape « vérité terrain », et extrait sur chacune des images de l'ensemble initial ;
- une étape d'apprentissage statistique automatique comprenant :
   - une sous-étape de sélection des images d'apprentissage parmi les images de l'ensemble initial ;
   - une sous-étape de tirage aléatoire des images d'apprentissage;
   - une sous-étape de réalisation automatique d'un classifieur numérique, par un calcul automatique d'une fonction de répartition à partir du ou des paramètre(s) numérique(s) calculé(s),
le classifieur numérique étant déterminé pour répartir automatiquement, à la fin de la sous-étape, les images d'apprentissage de l'ensemble initial en :
- un premier sous-groupe d'images d'apprentissage comportant le plus d'images dites avec visualisation «adéquate», et
- un deuxième sous-groupe d'images d'apprentissage comportant le plus d'images dites avec visualisation «inadéquate».

L'étape de « vérité terrain » peut être remplacée par une étape d'accès à une base de données déjà constituée d'un ensemble d'images réparties en images dites de qualité de visualisation « adéquate » et en images dites de qualité de visualisation « inadéquate » , en rapport à un ou plusieurs critères médicaux.

### Selon d'autres caractéristiques du procédé :

- les paramètres numériques sont choisis parmi la liste suivante:
   ∘ un paramètre colorimétrique global des images,
   ∘ un ou plusieurs paramètres reflétant l'abondance des bulles sur les images,
   ∘ un paramètre reflétant la luminosité de l'image qui est le contraste en niveaux de gris des images.
- le paramètre colorimétrique global de l'image est le ratio rouge/vert de l'image quand le segment digestif est l'intestin grêle ; ou le ratio rouge/(vert+bleu) quand le segment digestif est le côlon ;
- le paramètre reflétant l'abondance des bulles est :
   ∘ un paramètre textural issu de la matrice de co-occurrence en niveaux de gris (GLCM) de l'image traitée, ou
   ∘ une surface d'occupation des bulles
- le paramètre reflétant la luminosité des images est le contraste en niveaux de gris de l'image.
- il est calculé les trois paramètres numériques dans l'étape de calcul, et la sous-étape de réalisation automatique du classifieur numérique est réalisée avec les trois paramètres numériques.
- on répète plusieurs fois la sous- étape de réalisation automatique d'un classifieur numérique pour obtenir plusieurs classifieurs numériques.
- après l'étape d'apprentissage statistique automatique, on réalise :
   ∘ une étape de test numérique utilisant le ou les classifieurs appris automatiquement sur des images dites « de test » qui sont les images restantes de l'ensemble initial d'images moins les images d'apprentissage, dans le ou les classifieurs numériques, et
   ∘ une étape de décision numérique de la qualité de visualisation de chaque image de test, réalisée par le ou les classifieurs numériques.
- après l'étape d'apprentissage statistique automatique, on réalise :
   ∘ une étape de test numérique utilisant le ou les classifieurs appris automatiquement sur des images dites « de test » qui sont les images restantes de l'ensemble initial d'images moins les images d'apprentissage, dans le ou les classifieurs numériques, et
   ∘ une étape de décision numérique de la qualité de visualisation de chaque image de test, réalisée par le ou les classifieurs numériques.
- la fonction de répartition est une succession de seuils automatiques appliqués au ou aux paramètre(s) numérique(s) calculé(s),
- la sous-étape de réalisation du classifieur est découpée comme suit selon la technique dite « des forêts aléatoires » :
   ∘ plusieurs tirages aléatoires avec remise d'un même nombre d'images d'apprentissage sont réalisés, parmi le sous-ensemble des images d'apprentissage ;
   ∘ une analyse numérique de ces images d'apprentissage pour construire N arbres de décisions binaires,
   ∘ un arbre de décisions binaires par tirage aléatoire,
   ∘ chaque arbre de décisions binaires étant construit en utilisant en moins un des paramètres numériques,
   l'ensemble obtenu des arbres de décisions binaires constituant le classifieur numérique,
- l'étape de décision de la qualité de visualisation des images de test est un système de vote sur toutes les décisions des arbres de décisions binaires, chaque image de test ayant été testée dans tous les arbres de décisions binaires.
- la sous-étape de réalisation automatique du classifieur numérique est réalisée avec au moins deux paramètres numériques ;
- les seuils automatiques sont calculés automatiquement à chaque niveau de chaque arbre de décisions binaires avec le paramètre numérique qui permet la répartition des images en images avec visualisation «adéquate» et en images avec visualisation «inadéquate» la plus proche de la répartition des images avec visualisation «adéquate» et «inadéquate» réalisée lors de l'étape de « vérité terrain ».
- on répète la sous-étape de réalisation automatique du classifieur numérique, x fois, afin d'obtenir un classifieur constitué de l'ensemble des classifieurs numériques issus de l'étape d'apprentissage et donc ayant x*N arbres de décision binaire, avec N supérieur ou égal à 100 et x supérieur ou égal à 10.
- la sous-étape de réalisation du classifieur numérique est réalisée avec une technique choisie parmi la liste suivante :
   ∘ machine à vecteur de support ;
   ∘ des arbres de décision binaire en stratégie de boosting ;
   ∘ réseau de neurones.

Selon un autre aspect, l'invention porte sur un dispositif de réalisation d'un classifieur numérique d'images, afin de déterminer la qualité de visualisation des images de vidéocapsule endoscopique d'un segment du tube digestif, comprenant :
- une vidéocapsule permettant l'acquisition de vidéo de segments du tube digestif par une vidéocapsule ;
- des moyens de stockage des images, couplés à la vidéocapsule ;
- une base de données avec des images extraites de vidéocapsule et classées : en images dites avec visualisation «adéquate» et en images dites avec visualisation «inadéquate» ;
- des moyens de traitement et de calcul reliés aux moyens de stockage et intégrant la base de données, et configurés pour :
   - calculer au moins un paramètre numérique, qui a trait à l'un au moins des critères médicaux, et extrait des images de la base de données,
      le critère médical étant choisi parmi la liste suivante : le pourcentage de muqueuse visualisée, la luminosité de l'image, la présence de bulles, la présence de bile/chyme, la présence de liquides et de débris non digérés,
   - réaliser un apprentissage statistique automatique par :
      a) tirage aléatoire des images dites d'apprentissage, les images dites d'apprentissage étant choisies parmi les images de la base de données;
      b) réalisation automatique d'un classifieur numérique, par un calcul automatique d'une fonction de répartition à partir du ou des paramètre(s) numérique(s) calculé(s),
le classifieur numérique étant déterminé pour répartir automatiquement, à la fin de la sous-étape, les images d'apprentissage en un premier sous-groupe d'images d'apprentissage comportant le plus d'images dites avec visualisation «adéquate» et un deuxième sous-groupe d'images d'apprentissage comportant le plus d'images dites avec visualisation «inadéquate».

### Selon d'autres caractéristiques de l'invention, le dispositif selon l'invention :

- la sous-étape de réalisation automatique du classifieur numérique est réalisée avec les trois paramètres numériques suivants :
   ∘ le paramètre colorimétrique global de l'image est le ratio rouge/vert de l'image quand le segment digestif est l'intestin grêle ; ou le ratio rouge/(vert+bleu) quand le segment digestif est le côlon ;
   ∘ le paramètre reflétant l'abondance des bulles est :
      ▪ un paramètre textural issu de la matrice de co-occurrence en niveaux de gris (GLCM) de l'image traitée, ou
      ▪ une surface d'occupation des bulles
   ∘ le paramètre reflétant la luminosité des images est le contraste en niveaux de gris de l'image.
- les moyens de traitement et de calcul sont configurés pour :
   ∘ réaliser le classifieur numérique selon la technique dite « des forêts aléatoires » qui présente :
      ▪ plusieurs sous-étapes de tirage aléatoire avec remise d'un même nombre d'images d'apprentissage, parmi le sous-ensemble des images d'apprentissage ;
      ▪ une sous-étape d'analyse numérique de ces images d'apprentissage pour construire N arbres de décisions binaires,
      ▪ un arbre de décisions binaires par tirage aléatoire,
      ▪ chaque arbre de décisions binaires étant construit en utilisant en moins un des paramètres numériques,
      l'ensemble obtenu des arbres de décisions binaires constituant le classifieur numérique,
   ∘ -réaliser une étape de décision numérique de la qualité de visualisation des images de test, qui est un système de vote sur toutes les décisions numériques des arbres de décisions binaires, chaque image de test ayant été testée dans tous les arbres de décisions binaires.

Selon un troisième aspect, l'invention concerne un procédé de contrôle appliqué à une vidéo réalisée par vidéocapsule, d'au moins un segment du tube digestif d'une personne, pour déterminer automatiquement la qualité de visualisation des images de la vidéo, utilisant le classifieur numérique d'images réalisé par le procédé selon l'invention, appliqué aux images de la vidéo, pour déterminer automatiquement, lors d'un examen de contrôle automatique, les images avec visualisation «adéquate», les images avec visualisation «inadéquate», et le taux d'images avec visualisation «adéquate» dans la vidéo en fonction de la décision du classifieur numérique. Selon une autre caractéristique de l'invention, le procédé présente :
- une étape préalable de préparation intestinale à l'examen de contrôle, différente pour chaque personne ;
- une étape de contrôle de l'examen automatique,
- une étape de comparaison de l'efficacité des différentes préparations intestinales à l'examen en fonction du taux d'images avec visualisation « adéquate » déterminé pour chaque préparation intestinale différente par le procédé de contrôle.

Selon un quatrième aspect, l'invention propose un dispositif de contrôle pour déterminer automatiquement la qualité de visualisation d'une vidéo d'un ou de plusieurs segments du tube digestif d'une personne, réalisée par vidéocapsule endoscopique, comprenant:
- une vidéocapsule permettant l'acquisition de vidéo d'au moins un segment d'un tube digestif ;
- des moyens de stockage des images couplés à la vidéocapsule ;
- des moyens de traitement et de calcul reliés aux moyens de stockage et intégrant le classifieur numérique réalisé par le procédé défini selon l'invention,
et configurés pour :
- calculer au moins un paramètre numérique qui a trait à l'un au moins des critères médicaux, sur les images de la vidéo,
le critère médical étant choisi parmi la liste suivante : le pourcentage de muqueuse visualisée, la luminosité de l'image, la présence de bulles, la présence de bile/chyme, la présence de liquides et de débris non digérés
- tester numériquement les images de la vidéo, et décider numériquement des images avec visualisation «adéquate», des images avec visualisation «inadéquate», et du taux d'images avec visualisation «adéquate» dans la vidéo en fonction de la décision du classifieur numérique.

### DESCRIPTION DES FIGURES

D'autres objectifs, caractéristiques et avantages sortiront de la description détaillée qui suit en référence aux dessins donnés à titre illustratif et non limitatif parmi lesquels :
- la figure 1 représente des images correspondant à une note globale de score supérieure à 7 (image « propre ») ou < 7 (image « sale ») selon un mode de réalisation possible de l'invention ;
- la figure 2 représente la courbe ROC obtenue avec le procédé selon l'invention pour le paramètre ratio de pixels rouges/verts;
- la figure 3 représente la courbe ROC obtenue avec le procédé selon l'invention pour le paramètre GLCM;
- la figure 4 représente la courbe ROC obtenue avec le procédé selon l'invention pour le paramètre luminosité;

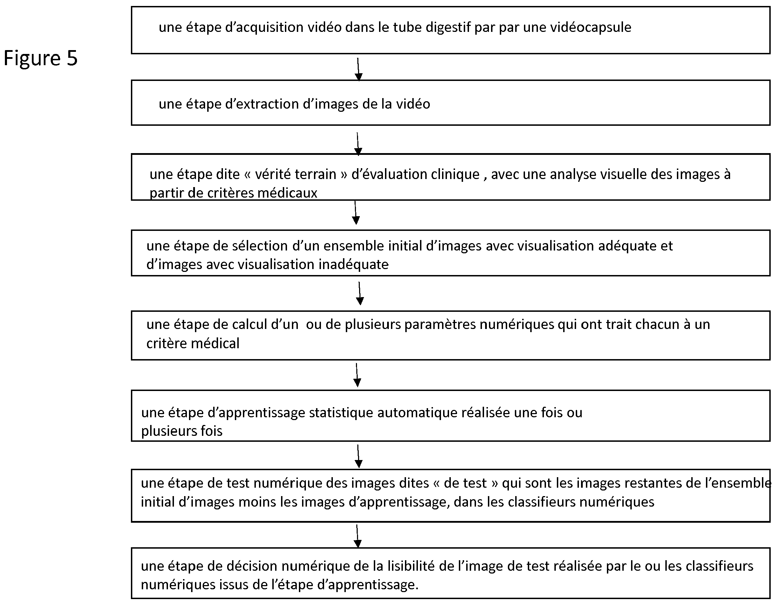

- la figure 5 représente le procédé selon l'invention ;

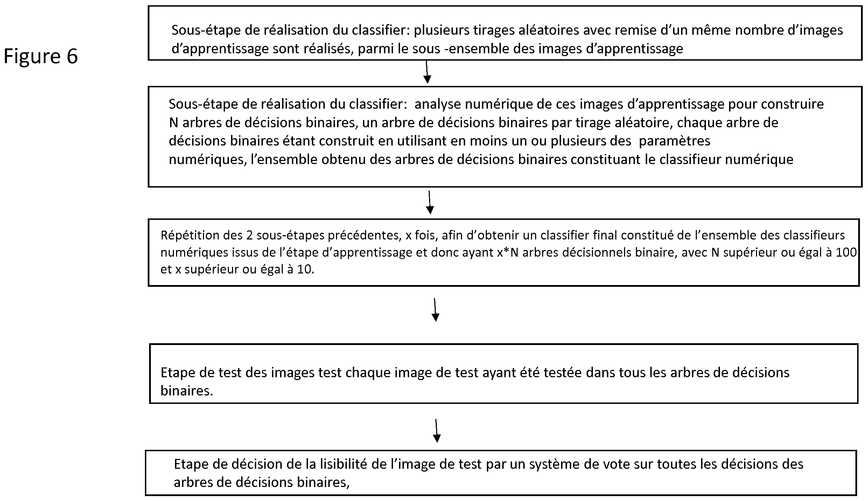

- la figure 6 représente la sous-étape de réalisation du classifieur selon un mode de réalisation de l'invention;
- la figure 7 représente l'explication de la méthode dite des forêts aléatoires ;
- la figure 8 représente les courbes ROC issues du procédé selon l'invention mettant en évidence l'amélioration des techniques d'analyse en utilisant les 3 paramètres.

### DESCRIPTION DETAILLEE DE L'INVENTION

### 1-Procédé de réalisation d'un classifieur numérique

La présente invention concerne un procédé de réalisation d'un classifieur numérique d'images, afin de déterminer automatiquement la qualité de visualisation des images de vidéocapsule endoscopique d'un ou de plusieurs segments du tube digestif.

Ce procédé de réalisation présente les étapes suivantes :
- une étape d'acquisition vidéo dans un ou plusieurs segments du tube digestif par une vidéocapsule ;
- une étape d'extraction d'images de la vidéo;
- une étape dite « vérité terrain » d'évaluation clinique de la qualité de visualisation des images à partir de critères médicaux, en déterminant un score par une analyse visuelle (réalisée par exemple par un médecin ou une personne compétente), pour répartir ces images, en fonction du résultat du score, en au moins : des images dites avec visualisation «adéquate» et des images dites avec visualisation «inadéquate» ;
- une étape de sélection d'un ensemble initial d'images parmi les images dites avec visualisation «adéquate» et les images dites avec visualisation «inadéquate»,
- une étape de calcul d'au moins un paramètre numérique qui a trait à l'un des critères médicaux de l'étape « vérité terrain », sur chacune des images.
- une étape d'apprentissage statistique automatique.
L'étape d'apprentissage statistique automatique comprend :
- une sous-étape de sélection des images d'apprentissage parmi les images de l'ensemble initial ;
- une sous-étape de tirage aléatoire des images d'apprentissage;
- une sous-étape de réalisation automatique d'un classifieur numérique des images d'apprentissage, à partir d'au moins un paramètre numérique, et qui est réalisée avec ou se base sur :
   - un calcul automatique d'une fonction de répartition à partir du ou des paramètres numériques calculés,
   - la répartition connue des images dites de qualité «adéquate» et en images dites de qualité «inadéquate» réalisée lors de l'étape « vérité terrain » ou connue d'une base de données.
La fonction de répartition peut être binaire ou en classes multiples.
Les critères médicaux peuvent être choisis notamment parmi la liste suivante : le pourcentage de muqueuse visualisée, la luminosité de l'image, la présence de bulles, la présence de bile/chyme, la présence de liquides et de débris non digérés (alimentaires, médicamenteux, fécaux, sang ou autres)
Les segments du tube digestif peuvent être l'intestin grêle, le côlon, l'oesophage et/ou l'estomac.
Avantageusement, on peut répéter la sous étape d'apprentissage automatique pour obtenir plusieurs classifieurs numériques.
Ensuite le procédé comporte:
- une étape de test numérique de chaque images dite « de test » (les images de « tests » sont les images restantes de l'ensemble initial d'images moins les images d'apprentissage), dans le ou les classifieurs numériques élaborés à l'étape d'apprentissage statistique automatique, et
- une étape de décision numérique de la qualité de visualisation de l'image de test réalisée par le ou les classifieurs numériques issus de l'étape d'apprentissage,
sont réalisées, et permettant de quantifier les performances (par exemple de déterminer une sensibilité et une spécificité).

Avantageusement, l'étape d'extraction d'images est réalisée à partir de vidéos de plusieurs personnes. Par exemple, on prend plusieurs centaines ou milliers images comme ensemble initial d'images E (par exemple 600), un sous-ensemble F dit « grand » de plusieurs centaines ou milliers d'images d'apprentissage (par exemple 500) et un sous-ensemble dit G « petit » des images de test avec G=E-F (par exemple 100).

Dans l'étape de sélection, il a été pris autant d'images d'apprentissage (par exemple 300) que d'images de test (par exemple 300).

Ce procédé peut être généralisé à l'oesophage, à l'estomac, à l'intestin grêle et/ou au côlon, individuellement ou de façon combinée (notamment grêle et côlon), avec l'utilisation de vidéocapsules endoscopiques adaptées à un ou plusieurs segments du tube digestif, selon les cas.

De même, il est possible d'utiliser des capsules panentériques qui couvrent toute la longueur du tube digestif.

Les analyses électroniques de qualité de visualisation pourront être réalisées en ligne sur Internet ; les vidéos peuvent être envoyées sur des serveurs, analysées en ligne, et les rapports renvoyés ensuite au médecin.

Le ou les paramètres numériques sont choisis parmi la liste suivante:
- un paramètre colorimétrique global de l'image, qui est par exemple le ratio rouge/vert de l'image pour l'intestin grêle ; ou le ratio rouge/(vert+bleu) pour le côlon ;
- un ou plusieurs paramètres reflétant l'abondance des bulles sur l'image, avec par exemple :
   - un paramètre textural issu de la matrice de co-occurrence en niveaux de gris (GLCM) de l'image traitée, ou
   - une surface d'occupation des bulles
- un paramètre reflétant la luminosité de l'image qui est le contraste en niveaux de gris de l'image (contraste tel que défini par Haralick [1974]).

Avantageusement, la sous-étape de réalisation automatique du classifieur numérique est réalisée avec les 3 paramètres numériques.

Avantageusement, on choisit le ou les paramètres numériques pour que le procédé présente une sensibilité (nombre d'images dites avec qualité de visualisation «adéquate» et sélectionnées par l'étape apprentissage / nombre total d'images dites avec qualité «adéquate») minimale de 90% et une spécificité (nombre d'images avec qualité de visualisation «inadéquate» et sélectionnées par l'étape d'apprentissage / nombre total d'images de visualisation «inadéquate» selon la vérité terrain) minimale de 70% voulues.

La présente invention concerne aussi un dispositif de réalisation d'un classifieur numérique d'images, afin de déterminer automatiquement la qualité de visualisation des images de vidéocapsule endoscopique de l'intestin grêle ou du côlon,
comprenant:
- une vidéocapsule permettant l'acquisition d'images du tube digestif par une vidéocapsule ;
- des moyens de stockage des images, couplés à la vidéocapsule ;
- une base de données avec des images extraites de vidéocapsule et classées : en images dites avec visualisation «adéquate» et en images dites avec visualisation «inadéquate» ;
- des moyens de traitement et de calcul reliés aux moyens de stockage et intégrant la base de données.

Ces moyens de traitement et de calcul sont configurés pour :
- calculer au moins un paramètre numérique qui a trait à au moins l'un des critères médicaux sur les images de la base de données, et ci-avant nommé,
- réaliser un apprentissage statistique automatique par :
   a) tirage aléatoire des images dites d'apprentissage, les images dites d'apprentissage étant choisies parmi les images de la base de données;
   b) réalisation automatique d'un classifieur numérique à partir d'au moins un paramètre numérique calculé, et qui est réalisé avec :
      - une définition automatique des seuils automatiques par rapport au paramètre numérique calculé,
      - la classification (ou répartition) des images en images «adéquate» et en images «inadéquate».

### 1.1) Technique dite « des forêts aléatoires »

Dans le cas où la fonction est binaire, la fonction de répartition est constituée d'une succession de seuils automatiques, et pour chaque « étage » de seuil, il est déterminé le paramètre numérique qui permet la meilleure répartition des images et le seuil automatique associé, ce paramètre numérique étant choisi (pouvant être différent) à chaque « étage » dans la succession des seuils automatiques pour optimiser la répartition des images.
Ainsi, les seuils automatiques sont déterminés pour répartir automatiquement, à la fin de la sous-étape d'apprentissage, les images d'apprentissage en deux sous-groupes, un premier sous-groupe d'images d'apprentissage devant comporter le plus d'images dites avec visualisation «adéquate» et un deuxième sous-groupe d'images d'apprentissage devant comporter le plus d'images dites avec visualisation «inadéquate».

En d'autres termes, la construction du classifieur est caractérisée par un calcul automatique et itératif et dichotomique de seuils associés aux paramètres numériques extraits des images d'apprentissage, pour répartir numériquement les images en images classées numériquement : en qualité «adéquate» ou en qualité «inadéquate».

Les seuils sont déterminés automatiquement dans l'étape d'apprentissage afin de minimiser l'erreur de classification (ou répartition) numérique des images classées numériquement (en qualité « adéquate » ou en qualité « inadéquate »), la vérité terrain des cliniciens servant ici de référence afin de pouvoir calculer l'erreur associée à un choix de seuil.

Les seuils ainsi appris automatiquement sont dits seuils finaux et sont ensuite utilisés dans l'étape de test pour classer numériquement des images dites « de test » et déterminer la sensibilité et la sélectivité [par rapport toujours aux images dont la répartition de référence (en qualité « adéquate » et en qualité « inadéquate ») est celle des cliniciens] de la méthode.

Plus précisément, la technique dite « des forêts aléatoires » est découpée comme suit:
- plusieurs tirages aléatoire avec remise d'un même nombre d'images d'apprentissage sont réalisés, parmi le sous -ensemble des images d'apprentissage ;
- une analyse numérique de ces images d'apprentissage pour construire N arbres de décisions binaires,
   un arbre de décisions binaires par tirage aléatoire,
   chaque arbre de décisions binaires étant construit en utilisant au moins un des paramètres numériques, et présentant à la fin de l'étape d'apprentissage des seuils automatiques calculés fonction des paramètres numériques
l'ensemble obtenu des arbres de décisions binaires constituant le classifieur numérique.

Les arbres de décision sont des outils de classification ou répartition appartenant à la catégorie des méthodes dites de partitionnement récursif. L'ensemble des observations est regroupé à la racine de l'arbre puis chaque division ou coupe sépare chaque noeud en deux noeuds fils plus *homogènes* que le noeud père au sens d'un *critère* à préciser. Dans le cas présent les paramètres R/V, GLCM, luminosité calculés sur chaque image d'apprentissage constitue un lot d'observation (variables explicatives) caractérisé par une vérité terrain (visualisation adéquate/inadéquate).

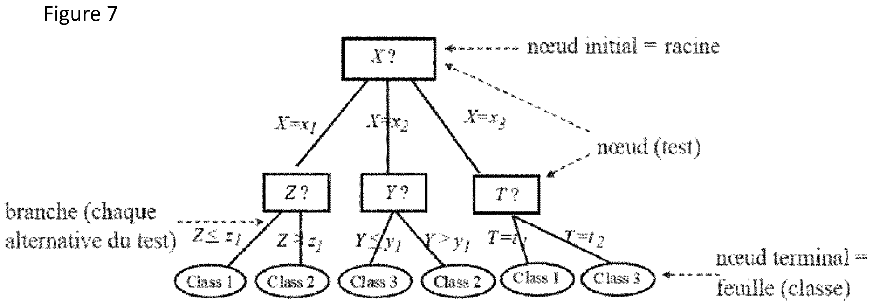

La construction d'un arbre de discrimination binaire (cf. figure 7) à partir de ces observations quantitatives consiste à déterminer une séquence de *noeuds :*
- Un noeud est défini par le choix conjoint d'une variable explicative et d'une *division* qui induit une partition en deux classes. Implicitement, à chaque noeud correspond donc un sous-ensemble de l'échantillon d'observation auquel est appliquée une dichotomie.
- Une division est elle-même définie par une *valeur seuil* de la variable explicative quantitative sélectionnée.
- À la racine ou noeud initial correspond l'ensemble de l'échantillon ; la procédure est ensuite itérée sur chacun des sous-ensembles.

L'algorithme nécessite :
- La définition d'un *critère* permettant de sélectionner la meilleure division parmi toutes celles *admissibles* pour les différentes variables (une division est dite *admissible* si aucun des deux noeuds descendants qui en découlent n'est vide ;
- Une règle permettant de décider qu'un noeud est terminal : il devient ainsi une *feuille* ;
- L'affectation de chaque feuille à l'une des classes ou à une valeur de la variable à expliquer (visualisation adéquate/inadéquate ici).

Le critère d'homogénéité permettant de sélectionner la meilleure division parmi celles admissible est dans le cas présent un critère entropique permettant de donner une mesure du gain de classification ou répartition entre deux niveaux de l'arbre (indice de Gini, entropie de Shannon).
Un ensemble d'arbres de décisions binaires construits sur des sous-ensembles issus des images d'apprentissage forme une forêt.
L'étape de décision de la qualité de visualisation des images de test peut être un système de vote sur toutes les décisions des arbres de décisions binaires, chaque image de test ayant été testée dans tous les arbres de décisions binaires.

### 1.2) Fonction de répartition multiclasses

Dans le cas où la fonction de répartition est multiclasses (nombre de classes >2), dans un autre mode de réalisation de l'invention, la sous-étape de réalisation du classifieur numérique est réalisée avec une technique choisie parmi la liste suivante :
- machine à vecteur de support ;
- réseau de neurones.
- des arbres de décision binaire en stratégie de boosting.

Le boosting est un domaine de l'apprentissage automatique (branche de l'intelligence artificielle). C'est un principe qui regroupe de nombreux algorithmes qui s'appuient sur des ensembles de classifieurs binaires : le boosting optimise leurs performances.

### 2-Procédé de contrôle

La présente invention concerne aussi un procédé de contrôle appliqué à une série d'images successives (vidéo) réalisée par vidéocapsule digestive (dans l'intestin grêle, côlon, l'estomac et/ou l'oesophage) d'une personne, pour déterminer numériquement la qualité de visualisation des images de la vidéo,
utilisant le classifieur numérique d'images réalisé par le procédé défini ci-avant,
appliqué aux images de la vidéo, pour déterminer numériquement les images avec visualisation «adéquate», les images avec visualisation «inadéquate», et le taux d'images «adéquate» dans la vidéo en fonction du résultat du classifieur numérique.

Dans ce cas :
- une étape de test numérique du classifieur numérique avec les seuils finaux est réalisée directement sur les images de la vidéo, et
- une étape de décision numérique de la qualité de visualisation est réalisée directement sur les images de la vidéo.
Il est ainsi possible :
- de mesurer objectivement l'efficacité de modalités de préparation intestinale à l'examen (par exemple des régimes alimentaires, des solutions laxatives ou des agents anti-bulles) ;
- de comparer objectivement l'efficacité de différentes modalités de préparation intestinale à l'examen (par exemple par des régimes alimentaires, des solution laxatives ou des agents anti-bulles) ;
- de décrire un niveau de qualité correcte de visualisation intestinale par vidéocapsule, avec uneproportion suffisante d'images à qualité de visualisation « adéquate » (par exemple plus de 80% des images à qualité de visualisation) permettant de conclure au caractère « propre » et « fiable » d'un examen par vidéocapsule, et sinon de renouveler cet examen.

Les protocoles de préparation intestinale validés permettront d'améliorer la qualité des soins, en évitant de refaire au même patient plusieurs vidéocapsules du fait d'une préparation et d'une visualisation intestinales inadéquates.

La présente invention concerne aussi un dispositif de contrôle pour déterminer numériquement et automatiquement la qualité de visualisation d'une vidéo de l'intestin grêle, du côlon, de l'estomac et/ou de l'oesophage) d'une personne, réalisée par vidéocapsule endoscopique, comprenant:
- une vidéocapsule permettant l'acquisition de vidéo d(un ou de plusieurs segments digestifs du tube digestif ;
- des moyens de stockage des images couplés à la vidéocapsule ;
- des moyens de traitement et de calcul reliés aux moyens de stockage et intégrant le classifieur numérique réalisé par le procédé défini ci-avant dans la description,

Les moyens de traitement et calcul sont configurés pour :
- Calculer au moins un paramètre numérique qui a trait à l'un au moins des critères médicaux, sur les images de la vidéo,
- Tester numériquement les images de la vidéo, et décider numériquement de la qualité de visualisation des images de la vidéo, avec le classifieur numérique réalisé selon le procédé défini ci-avant dans la description.

### PARTIE EXPERIMENTALE

L'objectif principal du procédé de l'invention est d'évaluer les performances diagnostiques, isolées puis combinées, de 3 méthodes d'analyse électronique de qualité de visualisation de la muqueuse d'images fixes de VCE du grêle de 3ème génération :
- l'analyse du ratio de pixels rouges/verts de l'image ;
- un indice reflétant l'abondance des bulles, basé sur une matrice de cooccurrence en niveaux de gris (GLCM) ;
- un indice reflétant la luminosité.

Il a été fait l'hypothèse première que dans une analyse électronique des images de VCE, combiner les approches "colorimétrie" et "détection de bulles" pourrait être plus performant qu'utiliser chaque approche isolément. De plus, en dehors de la colorimétrie (ratio rouges/verts, reflétant la visualisation de la muqueuse rose, et la présence de bile et résidus verdâtres) et de la quantité de bulles (seuil de 10%), les autres items du score de Brotz et al. (4) (la luminosité notamment) ne sont pas intégrés à l'analyse électronique des images. Il a donc été fait une hypothèse seconde qu'intégrer éventuellement en plus un paramètre reflétant le degré de luminosité des images de VCE pourrait améliorer encore leur analyse électronique en vue de l'évaluation de la qualité de visualisation de la muqueuse.

Les résultats sont concluants.

L'objectif secondaire a été de déterminer le temps nécessaire pour l'analyse d'une image selon le procédé de l'invention.

### Matériel et méthodes

### Sélection des patients et images

Trente vidéocapsules endoscopiques de l'intestin grêle complètes, normales de type Pillcam SB3® (Medtronic) étaient sélectionnées. Ces examens étaient tous réalisés dans le même centre hospitalier, après une préparation standardisée prescrite au patient. Ils étaient lus par un même praticien. L'indication était la même pour tous les examens : un saignement digestif inexpliqué. Toutes les vidéocapsules devaient être complètes (c'est-à-dire que l'intégralité de l'intestin grêle a été vu), et normales (aucune image pathologique n'était repérée, et aucune trace de sang n'était observée). La préparation globale de la VCE était qualifiée de « bonne », « « assez bonne » ou « moyenne ».
Les vidéos étaient anonymisées. Les séquences vidéo correspondant strictement à l'intestin grêle (de la première à la dernière image du grêle) étaient extraites et transformées en un format universel (mpeg) grâce au logiciel de lecture RAPID® (Medtronic).
Six cents images fixes étaient extraites de façon aléatoire et transformées en un format universel (jpeg), en vue de leur analyse électronique dans un second temps.
Ces images étaient lues par trois experts lecteurs de vidéocapsules endoscopiques du grêle. Les experts évaluaient les 600 images de façon indépendante, à l'insu de l'interprétation des autres lecteurs, et à l'insu de toute analyse électronique. Les experts notaient la qualité de l'image de chacune des 600 images selon la grille de l'indice quantitatif de Brotz et al. (4). Cinq critères étaient évalués :
- la pourcentage de muqueuse visualisée (< 80%, 80 à 90%, > 90%, accordant respectivement 0, 1 ou 2 points),
- la luminosité de l'image (importante notée à 0 point, modérée à 1 point et faible à 2 points),
- la présence de bulles (importante notée à 0 point, modérée à 1 point et faible à 2 points),
- la présence de bile/chyme (importante notée à 0 point, modérée à 1 point et faible à 2 points),
- la présence de liquides et de débris (importante, modérée ou faible, accordant respectivement 0, 1 ou 2 points).
Le score variait donc entre 0 et 10. La moyenne des trois notes constituait la « vérité terrain » *(groundtruth)* et servait de référence à l'évaluation des indices électroniques.
Les courbes de distribution du score quantitatif de l'article de Brotz et al. (4) étaient examinées. Il apparaissait que la grande majorité des séquences jugées "propres" dans cette étude avait un score quantitatif supérieur ou égal à 7. En conséquence, toute image de la banque dont le score quantitatif moyen était supérieur ou égal à 7 sur 10 était considérée comme ayant une "bonne" qualité de visualisation. Inversement, toute image dont le score quantitatif moyen était strictement inférieur à 7 sur 10 était considérée comme ayant une "mauvaise" qualité de visualisation.
Par extrapolation, pour chaque sous-score correspondant à chacun des 5 critères, il était considéré que l'analyse était "bonne" pour une valeur supérieure ou égale à 1,4 sur 2.

Ces images étaient analysées électroniquement selon les trois paramètres pré-cités (ratio de pixels rouges/verts, abondance des bulles, luminosité). Le résultat de l'analyse électronique était confronté à celui de la lecture experte, en termes de sensibilité et spécificité, pour les trois critères électroniques, individuellement, et combinés entre eux.

### Analyses électroniques et statistiques

Les 600 images étaient analysées électroniquement à l'Ecole Nationale Supérieure de l'Electronique et de ses Applications (ENSEA, Cergy) sur le logiciel MATLAB®. Pour chaque image étaient mesurés :
- le ratio de pixels rouges/verts,
- la présence de bulles grâce à une matrice de cooccurrence en niveaux de gris (GLCM),
- la luminosité ou contraste de l'image.

### 1-Etape d'apprentissage

Une première étape d'analyse électronique des images, dite d'apprentissage, utilisait une base constituée de 500 images tirées au sort parmi les 600 sélectionnées avec comme référence la « vérité terrain » établie par les lecteurs experts, afin d'entraîner le classifieur statistique considéré. Le classifieur choisi était développé à partir d'une méthode dite de forêts d'arbres décisionnels (aussi appelées forêts aléatoires) (11). Ce type d'outil d'aide à la décision est très largement utilisé dans le domaine de l'analyse de données par apprentissage.
Ces arbres de décision décrivent comment classer ou répartir un ensemble de données complexes (telles qu'une population ou, ici, une série d'images) selon un ensemble de variables discriminantes (ici ratio rouge/vert, matrice de cooccurrence, luminosité) et en fonction d'un objectif fixé en termes de nombre de classe (en l'occurrence, ici images de qualité de visualisation « bonne » ou "mauvaise") en s'appuyant sur une vérité terrain (ici les scores moyens des experts). Afin d'assurer une meilleure stabilité des performances de ce type d'algorithme (sensible à la base de données d'apprentissage), les forêts d'arbres décisionnels effectuent un apprentissage sur de multiples arbres de décision entraînés sur des sous-ensembles de données légèrement différents créés sur la base des 500 images considérées.

### 2-Etape de test

Une deuxième étape permettait de tester les performances du premier apprentissage.
Ici, la base de test était constituée de 100 images tirées au sort parmi les 600 images sélectionnées, avec comme référence la vérité terrain établie par les lecteurs experts. Ces images étaient classées par le classifieur précédemment appris. Il était alors possible d'en déduire les performances du classifieur.

### 3-Mesure des performances des classifieurs par "validation croisée"

Afin d'obtenir des performances statistiquement représentatives et non dépendantes du tirage des bases d'apprentissage et de test et de validation, une méthode de « validation croisée » était utilisée pour évaluer les performances diagnostiques des différents critères testés (ratio rouge/vert, bulles, luminosité, et leurs combinaisons). Cette méthode de validation croisée, éprouvée dans le domaine de l'analyse électronique d'images, reposait sur le principe suivant : un premier apprentissage était effectué sur 500 images tirées aléatoirement parmi les 600, puis la validation est faite sur les 100 images restantes ; l'opération était répétée dix fois. Les performances diagnostiques (sensibilité, spécificité) de la méthode étaient alors caractérisées par la courbe ROC moyenne obtenues sur les 10 tirages au sort (12).

### Critères de jugement

Le critère de jugement principal était la sensibilité et la spécificité de la combinaison des trois critères testés (ratio R/V, GLCM, luminosité).
Les critères de jugement secondaire étaient : la sensibilité et spécificité de chacun des critères pris individuellement.
Le temps nécessaire pour l'analyse électronique d'une image grâce aux 3 critères testés (ratio de pixels rouges/verts, contraste de matrice de co-ocurrence en niveaux de gris un indice de luminosité) était aussi testé.

### Résultats

### A) Analyse des experts

### A1) Analyse quantitative

Les notes moyennes (sur 2 points) attribuées pour chacun des critères par les 3 lecteurs-experts sont rapportées dans les tableaux 2 à 6. Les notes étaient inférieures au seuil de 1,4 sur 2 dans 72% des images pour le critère « pourcentage de muqueuse visualisée » (tableau 2), 65% pour le critère « débris, résidus, liquides » (tableau 3), 67% pour le critère « bulle » (tableau 4), 61% pour le critère « bile/chyme » (tableau 5) et 47% pour le critère « luminosité » (tableau 6). La note globale était supérieure ou égale à 7 sur 10, et donc permettait de répartir l'image comme ayant une "bonne" qualité, pour 40,5% des images pour le lecteur 1, 37% des images pour le lecteur 2, et 39% pour le lecteur 3, avec une moyenne de 37% pour les 3 lecteurs (tableau 7).

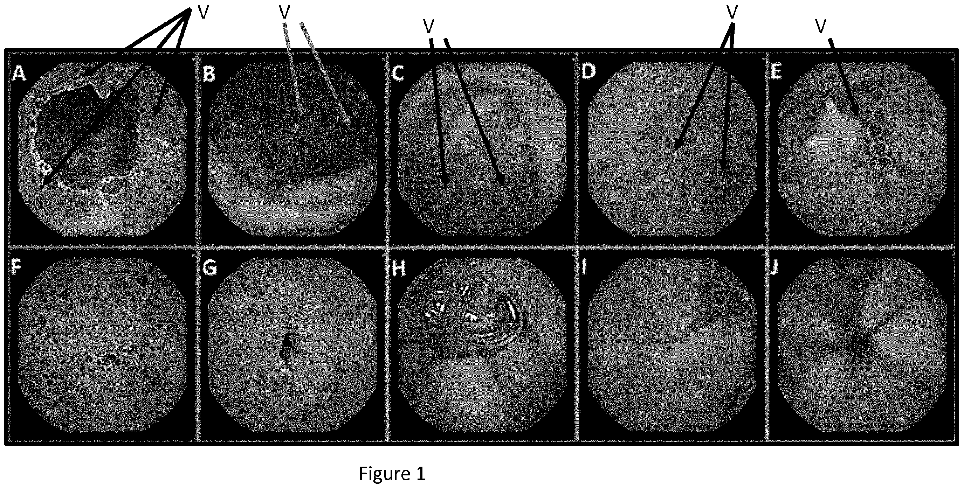

Des exemples d'images de qualité de préparation « bonnes » et « mauvaises » sont intégrés dans la Figure 1. Il a été indiqué par des flèches V les endroits qui sont verts (présence de bile) sur les photos

### A2) Corrélation inter-observateur

Les coefficients kappa de corrélation inter observateur sont indiqués dans le tableau 8 concernant la note globale, déterminant la propreté d'une image (note ≥ 7).
L'accord concernant l'analyse des trois experts était bon à excellent, le coefficient kappa du lecteur 1 avec le lecteur 2 et le lecteur 3 étant de 0,83 et 0,81 respectivement. Il était de 0,87 entre le lecteur 2 et le lecteur 3.

### B) Analyse électronique

### B1) Performances diagnostiques

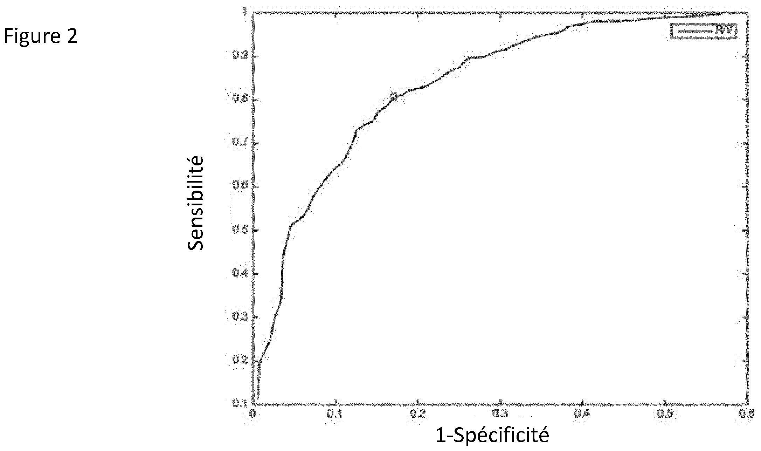

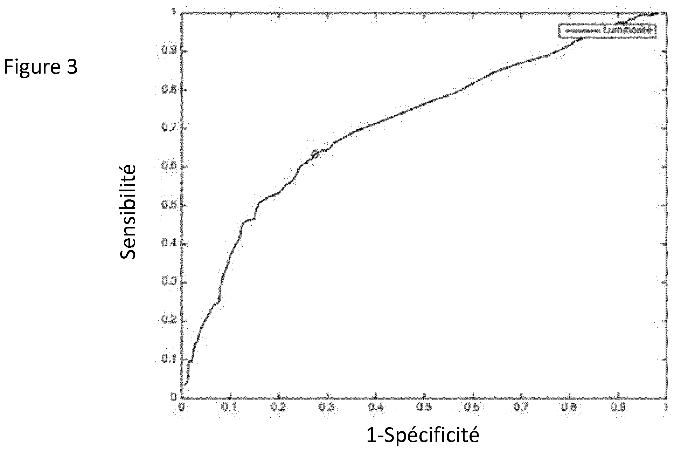

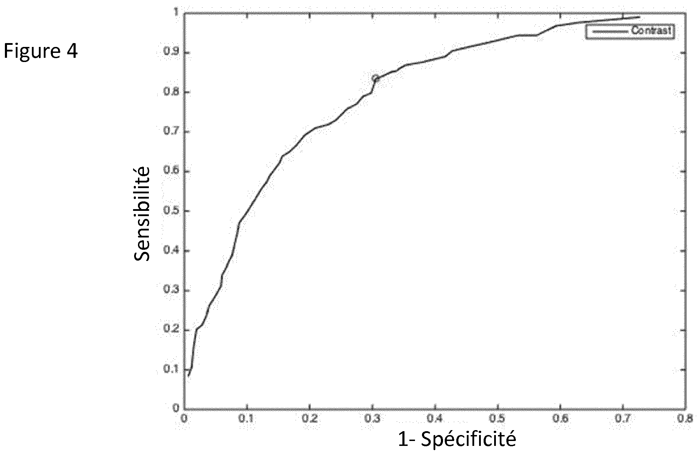

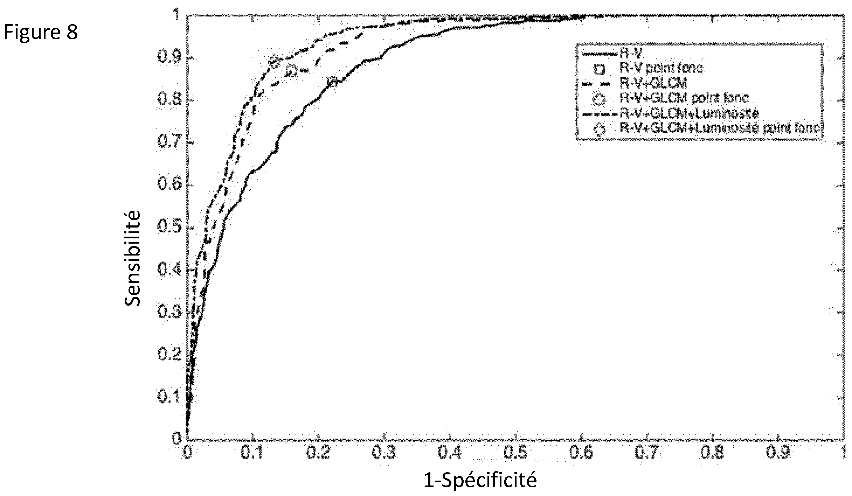

Les 600 images étaient analysées selon des algorithmes déjà validés (le ratio de pixels rouge/vert, une méthode d'évaluation des contrastes issue de la matrice de cooccurrence, le contraste de l'image s'apparentant à la luminosité de celle-ci) et comparées à la lecture des trois experts, en ayant déterminé que le seuil au-delà duquel une image était de bonne qualité de visualisation avait une note globale supérieure ou égale à 7 sur 10.
Concernant le critère ratio de pixels rouges/verts, la sensibilité était de 80% et la spécificité de 83% (Figure 2).
Pour le contraste d'une matrice de cooccurrence en niveaux de gris, la sensibilité et la spécificité étaient respectivement de 84% et 70% (Figure 4).
Pour la luminosité, la sensibilité était de 62% et la spécificité de 74% (Figure 3).
Grâce à la méthode des arbres de régression permettant une combinaison de ces trois critères, la sensibilité était de 90% et la spécificité de 88% (Figure 8).

### B2) Temps d'analyse

Le temps nécessaire à l'analyse électronique d'une image selon les trois critères testés (ratio de pixels rouges/verts, contraste de matrice de co-ocurrence en niveaux de gris un indice de luminosité) était de 2,3 millisecondes.

### B3) Discussion

Il a été proposé selon l'invention une méthode d'analyse électronique composite permettant de déterminer si une image fixe de VCE de l'intestin grêle présente une bonne qualité de visualisation. Cette méthode inclut trois paramètres: le ratio de pixels rouges/verts (représentant la visualisation muqueuse), un contraste de matrice de cooccurrence en niveaux de gris (représentant l'abondance des bulles) et un indice de luminosité. La combinaison brute de ces trois critères permet d'obtenir de bonnes performances diagnostiques : sensibilité à 90% et spécificité à 88%.

L'échelle nous paraissant la plus complète, fiable, reproductible et fiable afin de conclure à la qualité de visualisation des images de l'examen est l'échelle quantitative de Brotz et al. (4) sur laquelle nous nous sommes basés ici pour l'évaluation des experts. Parmi les trois scores développés par les auteurs, l'échelle quantitative est celle ayant la meilleure reproductibilité (comparée à l'échelle qualitative et l'évaluation globale) avec des coefficients intra observateurs cependant modérés (0,60 à 0,66) sur des séquences vidéos.

La plupart des travaux antérieurs analyse l'ensemble de l'examen ou des séquences vidéos afin de déterminer si la qualité de visualisation est satisfaisante. En effet, l'analyse de vidéos peut être moins précise car évaluant des séquences de milliers d'images, souvent hétérogènes en termes de qualité de visualisation, amenant donc à un jugement moins reproductible. Afin d'obtenir une vérité terrain solide, il a été décidé de baser le travail sur l'évaluation d'images fixes dans l'objectif de l'intégrer à un logiciel informatique, l'Homme ne pouvant, à terme, pas analyser des images une par une. Une analyse électronique indépendante de l'oeil humain serait objective, rapide, parfaitement reproductible, et permet un gain de temps considérable.

Au total, pour établir une "vérité terrain", il a été donc considéré 600 images fixes (et non des séquences contenant des milliers d'images) et il a été utilisé la moyenne des scores quantitatifs de trois experts (et non des scores individuels). Ce score quantitatif, proposé et publié par Brotz et al. (4) a été utilisé depuis sa publication dans d'autres travaux. Les lectures d'experts étaient réalisées pour chaque expert à l'insu de l'interprétation des autres lecteurs, et à l'insu de toute analyse électronique.

Le travail a évalué un score des plus complets, intégrant de multiples composantes qualifiant la qualité de visualisation d'une image de VCE du grêle : le pourcentagede muqueuse visualisée, l'abondance des bulles, la présence de bile, résidus, débris, et la luminosité. La « vérité terrain » est basée sur un fort accord entre l'analyse des trois experts, les coefficients kappa allant de 0,81 à 0,87 concernant la note globale.

Les points forts sont qu'il a été évalué une base de données d'images large et variée, que nos résultats se basent sur une « vérité terrain » solide avec des accords inter observateur forts. Il s'agit d'un travail se basant sur une analyse électronique donc objective, reproductible qui pourrait à terme, si elle aboutit à un score validé, être intégrée à des logiciels de lecture de VCE. Le travail, original, est le premier proposant un score aussi complet, prenant en compte de multiples critères de qualité d'évaluation de la qualité de visualisation des images en VCE de l'intestin grêle.

### Conclusion

Il est proposé une méthode d'évaluation électronique multicritère intégrant l'analyse d'un ratio de pixels rouges/verts, l'abondance des bulleset la luminosité de l'image afin de déterminer la qualité de visualisation des images en VCE de l'intestin grêle de 3ème génération. Ce score composite est fiable, facile d'utilisation, reproductible et pourrait être intégré à des logiciels de lecture afin de faciliter la détermination de la qualité de visualisation des images en VCE et ainsi de conclure au niveau de fiabilité de chaque examen. Il pourrait aussi permettre de comparer de manière reproductible les différentes méthodes de préparation afin de valider la procédure à suivre et les recommandations.

### Tableaux

**Tableau 1 : Grille d'évaluation des images**

| **Points** | **% de muqueuse visualisée** | **Quantité de débris/résidus/ fluide** | **Quantité de bulles** | **Quantité de chyme/bile** | **Réduction de la luminosité** |
|---|---|---|---|---|---|
| 0 | <80% | importante | importante | importante | importante |
| 1 | 80-89% | modérée | modérée | modérée | modérée |
| 2 | >= 90% | minime/faible | minime/faible | minime/faible | minime/faible |

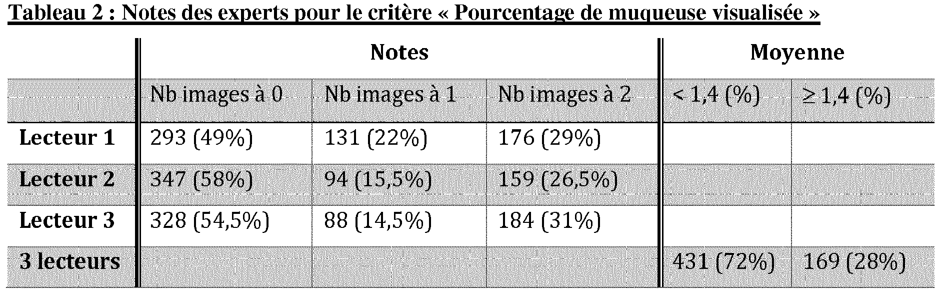

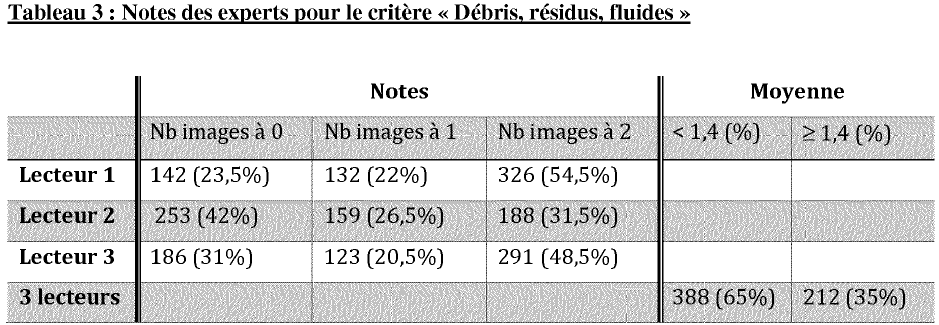

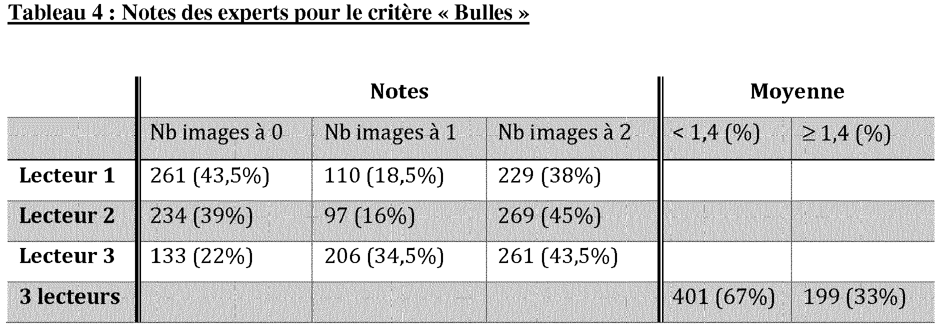

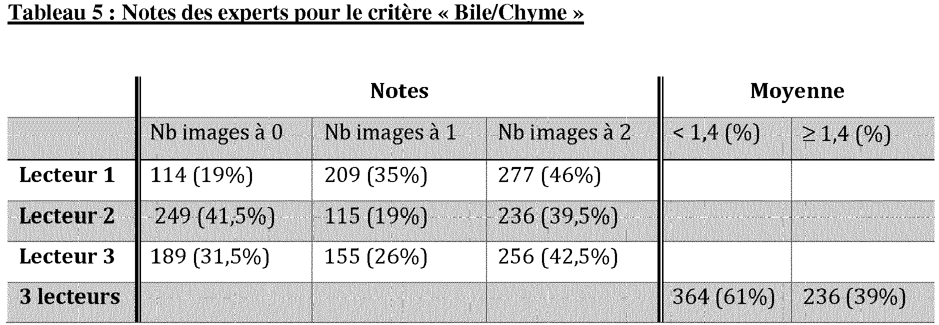

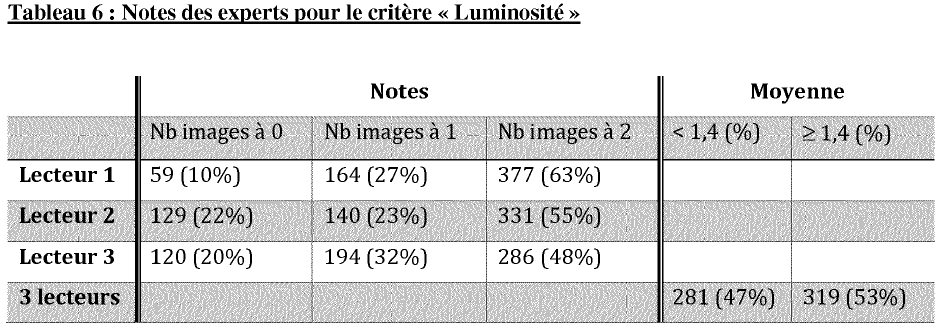

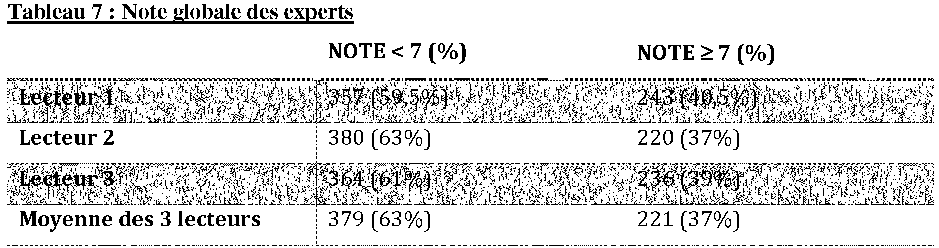

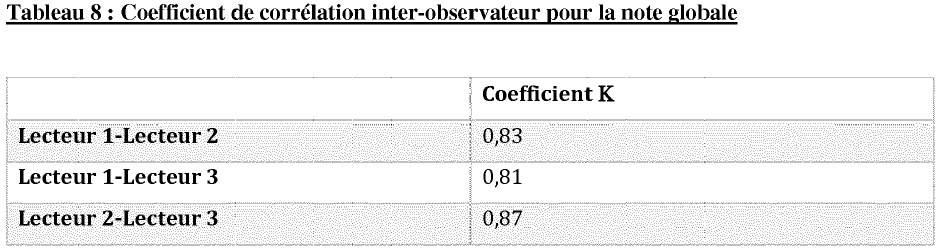

**Tableau 9: Performances diagnostiques de l'analyse informatisée pour discriminer les images fixes «adéquate» et «inadéquate»**

| **Paramètres numériques** | **Sensibilité %, [I.C._{95%}]** | **Spécificité %, [I.C._{95%}]** |
|---|---|---|
| ratioR/V | 84,06 [76,89; 91,23] | 78,67 [70,64; 86,70] |
| Abondance de bulles | 79,61 [71,71; 87,51] | 73,60 [64,96; 82,24] |
| Luminosité | 73,96 [65,36; 82,56] | 78,37 [70,30; 86,44] |
| ratio R/V+ abondance de bulles | 85,24 [78,29; 92,19] | 86,36 [79,63; 93,09] |
| Abondance de bulles + luminosité | 85,20 [78,24; 92,16] | 78,98 [70,99; 86,97] |
| ratio R/V + luminosité | 86,12 [76,34; 92,90] | 86,20 [79,44; 92,96] |
| ratio R/V + abondance de bulles + luminosité | 90,01 [84,12; 95,88] | 87,73 [81,30; 94,16] |

### Références

1. McAlindon ME, Ching H-L, Yung D, Sidhu R, Koulaouzidis A. Capsule endoscopy of the small bowel. Ann Transi Med. 2016;4(19):369.
2. Rokkas T, Papaxoinis K, Triantafyllou K, Pistiolas D, Ladas SD. Does purgative préparation influence the diagnostic yield of small bowel video capsule endoscopy?: A meta-analysis. Am J Gastroenterol. 2009;104(1):219- 27.
3. Ladas SD, Triantafyllou K, Spada C, Riccioni ME, Rey J-F, Niv Y, Delvaux M, de Franchis R, Costamagna G; ESGE Clinical Guidelines Committee. European Society of Gastrointestinal Endoscopy (ESGE): recommendations (2009) on clinical use of video capsule endoscopy to investigate small-bowel, esophageal and colonie diseases. Endoscopy. 2010;42(3):220- 7.
4. Brotz C, Nandi N, Conn M, Daskalakis C, DiMarino M, Infantolino A, Katz LC, Schroeder T, Kastenberg D. A validation study of 3 grading systems to evaluate small-bowel cleansing for wireless capsule endoscopy: a quantitative index, a qualitative evaluation, and an overall adequacy assessment. Gastrointest Endosc. 2009;69(2):262- 270, 270.e1.
5. Albert J, Göbel C-M, Lesske J, Lotterer E, Nietsch H, Fleig WE. Simethicone for small bowel préparation for capsule endoscopy: a systematic, single-blinded, controlled study. Gastrointest Endosc. 2004;59(4):487- 91.
6. Ninomiya K, Yao K, Matsui T, Sato Y, Kishi M, Karashima Y, Ishihara H, Hirai F . Effectiveness of magnésium citrate as préparation for capsule endoscopy: a randomized, prospective, open-label, inter-group trial. Digestion. 2012;86(1):27-33.
7. Goyal J, Goel A, McGwin G, Weber F. Analysis of a grading system to assess the quality of small-bowel préparation for capsule endoscopy: in search of the Holy Grail. Endosc Int Open. 2014;2(3):E183-186.
8. Park SC, Keum B, Hyun JJ, Seo YS, Kim YS, Jeen YT, Chun HJ, Um SH, Kim CD, Ryu HS. A novel cleansing score system for capsule endoscopy. World J Gastroenterol. 2010;16(7):875- 80.
9. Van Weyenberg SJB, De Leest HTJI, Mulder CJJ. Description of a novel grading system to assess the quality of bowel préparation in video capsule endoscopy. Endoscopy. 2011;43(5):406- 11.
10. Abou Ali E, Histace A, Camus M, Gerometta R, Becq A, Nion-Larmurier I, Ulriikka Chaput, Philippe Marteau, Olivier Romain, Christian Florent, Xavier Dray. Development and Validation of a Highly Sensitive and Highly Specific Computed Assessment of Cleansing Score for Small Bowel Capsule Endoscopy. United European Gastroenterology Week. 2016;
11. Moutarde F. Arbres de Décision et Forêts Aléaoires. 2017.
12. Kohavi R. A Study of Cross-validation and Bootstrap for Accuracy Estimation and Model Selection. In: Proceedings of the 14th International Joint Conférence on Artificial Intelligence - Volume 2. San Francisco, CA, USA: Morgan Kaufmann Publishers Inc.; 1995. p. 1137-1143. (IJCAI'95).

## Revendications

1. Procédé de réalisation d'un classifieur numérique d'images déterminant automatiquement la qualité de visualisation des images de vidéocapsule endoscopique dans au moins un segment du tube digestif d'une personne,
comprenant les étapes suivantes :
- une étape d'acquisition vidéo dans le segment du tube digestif par une vidéocapsule ;
- une étape d'extraction d'images de la vidéo;
- une étape dite « vérité terrain » d'évaluation clinique de la qualité de visualisation des images par un score, déterminé par une analyse visuelle des images, à partir d'un ou plusieurs critères médicaux,
pour répartir ces images, en fonction du résultat du score : en images dites avec visualisation «adéquate» et en images dites avec visualisation «inadéquate» ;
les critères médicaux étant choisis parmi la liste suivante : le pourcentage de muqueuse visualisée, la luminosité de l'image, l'abondance des bulles, la présence de bile/chyme, la présence de liquides et de débris non digérés,
- une étape de sélection d'un ensemble initial d'images parmi les images dites avec visualisation «adéquate» et les images dites avec visualisation «inadéquate»,
- une étape de calcul d'au moins un paramètre numérique qui a trait à au moins l'un des critères médicaux de l'étape « vérité terrain », et extrait sur chacune des images de l'ensemble initial ;
- une étape d'apprentissage statistique automatique comprenant :
• une sous-étape de sélection des images d'apprentissage parmi les images de l'ensemble initial ;
• une sous-étape de tirage aléatoire des images d'apprentissage;
• une sous-étape de réalisation automatique d'un classifieur numérique, par un calcul automatique d'une fonction de répartition à partir du ou des paramètre(s) numérique(s) calculé(s),
le classifieur numérique étant déterminé pour répartir automatiquement, à la fin de la sous-étape, les images d'apprentissage de l'ensemble initial en :
- un premier sous-groupe d'images d'apprentissage comportant le plus d'images dites avec visualisation «adéquate», et
- un deuxième sous-groupe d'images d'apprentissage comportant le plus d'images dites avec visualisation «inadéquate».

2. Procédé selon la revendication 1, **caractérisé en ce que** le ou les paramètres numériques sont choisis parmi la liste suivante:
- un paramètre colorimétrique global des images,
- un ou plusieurs paramètres reflétant l'abondance des bulles sur les images,
- un paramètre reflétant la luminosité de l'image qui est le contraste en niveaux de gris des images.

3. Procédé selon la revendication 2, dans lequel :
- le paramètre colorimétrique global de l'image est le ratio rouge/vert de l'image quand le segment digestif est l'intestin grêle ; ou le ratio rouge/(vert+bleu) quand le segment digestif est le côlon ;
- le paramètre reflétant l'abondance des bulles est :
• un paramètre textural issu de la matrice de co-occurrence en niveaux de gris (GLCM) de l'image traitée, ou
• une surface d'occupation des bulles
- le paramètre reflétant la luminosité des images est le contraste en niveaux de gris de l'image.

4. Procédé selon la revendication 3, dans lequel il est calculé les 3 paramètres numériques dans l'étape de calcul, et la sous-étape de réalisation automatique du classifieur numérique est réalisée avec les 3 paramètres numériques.

5. Procédé selon l'une des revendications 1 à 4, dans lequel :
on répète plusieurs fois la sous- étape de réalisation automatique d'un classifieur numérique pour obtenir plusieurs classifieurs numériques.

6. Procédé selon l'une des revendications 1 à 5, dans lequel après l'étape d'apprentissage statistique automatique, on réalise :
- une étape de test numérique utilisant le ou les classifieurs appris automatiquement sur des images dites « de test » qui sont les images restantes de l'ensemble initial d'images moins les images d'apprentissage, dans le ou les classifieurs numériques, et
- une étape de décision numérique de la qualité de visualisation de chaque image de test, réalisée par le ou les classifieurs numériques.

7. Procédé selon l'une des revendications 1 à 6, dans lequel la fonction de répartition est une succession de seuils automatiques appliqués au ou aux paramètre(s) numérique(s) calculé(s).

8. Procédé selon la revendication 7, dans lequel :
- la sous-étape de réalisation du classifieur est découpée comme suit selon la technique dite « des forêts aléatoires » :
• plusieurs tirages aléatoires avec remise d'un même nombre d'images d'apprentissage sont réalisés, parmi le sous-ensemble des images d'apprentissage ;
• une analyse numérique de ces images d'apprentissage pour construire N arbres de décisions binaires,
un arbre de décisions binaires par tirage aléatoire,
chaque arbre de décisions binaires étant construit en utilisant en moins un des paramètres numériques,
l'ensemble obtenu des arbres de décisions binaires constituant le classifieur numérique,
- l'étape de décision de la qualité de visualisation des images de test est un système de vote sur toutes les décisions des arbres de décisions binaires, chaque image de test ayant été testée dans tous les arbres de décisions binaires.

9. Procédé selon la revendication 8, dans lequel :
- la sous-étape de réalisation automatique du classifieur numérique est réalisée avec au moins deux paramètres numériques ;
- les seuils automatiques sont calculés automatiquement à chaque niveau de chaque arbre de décisions binaires avec le paramètre numérique qui permet la répartition des images en images avec visualisation «adéquate» et en images avec visualisation «inadéquate» la plus proche de la répartition des images avec visualisation «adéquate» et «inadéquate» réalisée lors de l'étape de « vérité terrain ».

10. Procédé selon la revendication 9, dans lequel :
- on répète la sous-étape de réalisation automatique du classifieur numérique, x fois, afin d'obtenir un classifieur final constitué de l'ensemble des classifieurs numériques issus de l'étape d'apprentissage et donc ayant x*N arbres de décision binaire, avec N supérieur ou égal à 100 et x supérieur ou égal à 10.

11. Procédé selon l'une des revendications 1 à 6, dans lequel :
la sous-étape de réalisation du classifieur numérique est réalisée avec une technique choisie parmi la liste suivante :
- machine à vecteur de support ;
- des arbres de décision binaire en stratégie de boosting ;
- réseau de neurones.

12. Dispositif de réalisation d'un classifieur numérique d'images, afin de déterminer la qualité de visualisation des images de vidéocapsule endoscopique d'un segment du tube digestif, comprenant:
- une vidéocapsule permettant l'acquisition de vidéo de segments du tube digestif par une vidéocapsule ;
- des moyens de stockage des images, couplés à la vidéocapsule ;
- une base de données avec des images extraites de vidéocapsule et classées : en images dites avec visualisation «adéquate» et en images dites avec visualisation «inadéquate» ;
- des moyens de traitement et de calcul reliés aux moyens de stockage et intégrant la base de données, et configurés pour :
• calculer au moins un paramètre numérique, qui a trait à l'un au moins des critères médicaux, et extrait des images de la base de données,
le critère médical étant choisi parmi la liste suivante : le pourcentage de muqueuse visualisée, la luminosité de l'image, la présence de bulles, la présence de bile/chyme, la présence de liquides et de débris non digérés,
• réaliser un apprentissage statistique automatique par :
a) tirage aléatoire des images dites d'apprentissage, les images dites d'apprentissage étant choisies parmi les images de la base de données;
b) réalisation automatique d'un classifieur numérique, par un calcul automatique d'une fonction de répartition à partir du ou des paramètre(s) numérique(s) calculé(s),
le classifieur numérique étant déterminé pour répartir automatiquement, à la fin de la sous-étape, les images d'apprentissage en un premier sous-groupe d'images d'apprentissage comportant le plus d'images dites avec visualisation «adéquate» et un deuxième sous-groupe d'images d'apprentissage comportant le plus d'images dites avec visualisation «inadéquate».

13. Dispositif selon la revendication 12, dans lequel la sous-étape de réalisation automatique du classifieur numérique est réalisée avec les 3 paramètres numériques suivants :
- le paramètre colorimétrique global de l'image est le ratio rouge/vert de l'image quand le segment digestif est l'intestin grêle ; ou le ratio rouge/(vert+bleu) quand le segment digestif est le côlon ;
- le paramètre reflétant l'abondance des bulles est :
• un paramètre textural issu de la matrice de co-occurrence en niveaux de gris (GLCM) de l'image traitée, ou
• une surface d'occupation des bulles
- le paramètre reflétant la luminosité des images est le contraste en niveaux de gris de l'image.

14. Dispositif selon l'une des revendications 12 à 13, dans lequel :
les moyens de traitement et de calcul sont configurés pour :
- réaliser le classifieur numérique selon la technique dite « des forêts aléatoires » qui présente :
• plusieurs sous-étapes de tirage aléatoire avec remise d'un même nombre d'images d'apprentissage, parmi le sous-ensemble des images d'apprentissage ;
• une sous-étape d'analyse numérique de ces images d'apprentissage pour construire N arbres de décisions binaires,
un arbre de décisions binaires par tirage aléatoire,
chaque arbre de décisions binaires étant construit en utilisant en moins un des paramètres numériques,
l'ensemble obtenu des arbres de décisions binaires constituant le classifieur numérique,
- réaliser une étape de décision numérique de la qualité de visualisation des images de test, qui est un système de vote sur toutes les décisions numériques des arbres de décisions binaires, chaque image de test ayant été testée dans tous les arbres de décisions binaires.

15. Procédé de contrôle appliqué à une vidéo réalisée par vidéocapsule, d'au moins un segment du tube digestif d'une personne, pour déterminer automatiquement la qualité de visualisation des images de la vidéo,
utilisant le classifieur numérique d'images réalisé par le procédé selon l'une des revendications 1 à 11, appliqué aux images de la vidéo,
pour déterminer automatiquement, lors d'un examen de contrôle automatique, les images avec visualisation «adéquate», les images avec visualisation «inadéquate», et le taux d'images avec visualisation «adéquate» dans la vidéo en fonction de la décision du classifieur numérique.

16. Procédé de contrôle selon la revendication 15, appliqué à différentes personnes, **caractérisé en ce que** le procédé présente :
- une étape préalable de préparation intestinale à l'examen de contrôle, différente pour chaque personne ;
- une étape de contrôle de l'examen automatique,
- une étape de comparaison de l'efficacité des différentes préparations intestinales à l'examen en fonction du taux d'images avec visualisation « adéquate » déterminé pour chaque préparation intestinale différente par le procédé de contrôle.

17. Dispositif de contrôle pour déterminer automatiquement la qualité de visualisation d'une vidéo d'un ou de plusieurs segments du tube digestif d'une personne, réalisée par vidéocapsule endoscopique, comprenant:
- une vidéocapsule permettant l'acquisition de vidéo d'au moins un segment d'un tube digestif ;
- des moyens de stockage des images couplés à la vidéocapsule ;
- des moyens de traitement et de calcul reliés aux moyens de stockage et intégrant le classifieur numérique réalisé par le procédé défini selon l'une des revendications 1 à 11,
et configurés pour :
• calculer au moins un paramètre numérique qui a trait à l'un au moins des critères médicaux, sur les images de la vidéo,
le critère médical étant choisi parmi la liste suivante : le pourcentage de muqueuse visualisée, la luminosité de l'image, la présence de bulles, la présence de bile/chyme, la présence de liquides et de débris non digérés
• tester numériquement les images de la vidéo, et décider numériquement des images avec visualisation «adéquate», des images avec visualisation «inadéquate», et du taux d'images avec visualisation «adéquate» dans la vidéo en fonction de la décision du classifieur numérique.
